# EUROPEAN PATENT APPLICATION

(11) **EP 2 423 314 A1**
(43) Date of publication of application: **29.02.2012**
(21) Application number: 11190840.6
(22) Date of filing: 03.08.2007
(51) Int. Cl.: C12N 15/64, C12N 15/66, C12N 15/70, C12N 15/10, A61K 39/00

(54) **Vector systems**

(30) Priority: 03.08.2006 US 821339 P
(62) Divisional of application: 07840694.9
(71) Applicant: Wisconsin Alumni Research Foundation, Madison, WI 53705 (US)
(72) Inventor: Szybalski, Waclaw, Madison, WI 53705 (US); Wild, Jadwiga, Vero Beach, FL Florida 32963 (US); Hradecba, Zdenka, Madison, WI 53705 (US); Frisch, David, Madison, WI 53711 (US); Blattner, Frederick, Madison, WI 53711 (US); Gromek, Katarzyna, Madison, WI 53705 (US)
(74) Representative: den Hartog, Jeroen H.J.

(57) **Abstract**

The present invention relates to the cloning of nucleic acid molecules and the production of nucleic acid libraries, as well as the expression of recombinant proteins and bactofection. More in particular, the invention relates to a method of inducing and expressing nucleic acid in an animal cell, the method comprising: (a) providing a vector comprising an insertion site, a first origin of replication a second origin of replication, and a pair of transcriptional terminators; (b) introducing the nucleic acid into the insertion site of the vector; (c) transforming at least one invasive bacterium with the vector to form at least one transformed bacterium; and (d) infecting the animal cell with said at least one transformed bacterium.

## Description

### BACKGROUND OF THE INVENTION

### Field of the Invention

The present invention relates generally to the field of molecular biology and genomics. More specifically, the present invention concerns the cloning of nucleic acid molecules and the production of nucleic acid libraries, as well as the expression of recombinant proteins and bactofection.

### 1. Description of Related Art

The impacts of recombinant DNA, recombinant protein and genomics in biomedical research and therapeutic treatments have been major. These methods require sequence information, which in turn requires high quality genomic libraries representing the entire genome with minimal bias. DNA fragments for genomic libraries are often produced using restriction enzymes to partially digest the genome, which has several limitations. Partial digestion of the genome leads to bias against regions of the genome where the distance between restriction sites is less than or greater than the applied size-selection limits. As an alternative to the cloning bias associated with partial digestion, DNA fragments can be produced by randomly shearing the genome. However, cloning efficiencies are significantly reduced using randomly sheared DNA.

The bacterial artificial chromosome (BAC) vectors were developed for the construction and faithful maintenance of genomic libraries in bacteria. BAC vectors are based on the F plasmid, which maintains the vector at 1-2 copies per cell. Low copy number is an important feature for clone stability by limiting the amount of homologous sequence subject to recombination. However, the crucial disadvantage of low copy number is that it makes both preparing the vector for cloning and downstream analyses of clones, such as by sequencing and fingerprinting, more costly and laborious.

Medium- to high-copy number plasmids are often preferred over the low- or single-copy number plasmids for over-production of recombinant DNA and protein, because they typically lead to high yields of the target products. However, over-production of certain targets may cause unwanted toxicity to the cell, impose a significant metabolic stress on the host, and cause severe growth retardation. Bacterial hosts often respond to the over-production of "unwanted" material by naturally selecting those mutated cells that have drastically reduced, or completely eliminated, the production of the "toxic" product.

What the art needs are vectors that allow the construction of high quality genomic libraries that are able to faithfully represent an entire genome as well as providing the ability to propagate, map, express and analyze cloned material with little or no need to re-clone into a different vector. The genomic libraries, clones and subclones should be produced efficiently and with high yield. The genomic libraries should also be easily propagated and analyzed. The art also needs improved vectors for production of recombinant DNA or protein.

If these and other needs could be addressed, a significant advance in the art would result.

### Summary of the Invention

In one embodiment, a vector may comprise an excisable fragment comprising an insertion site, a first and second origin of replication, and a pair of transcriptional terminators flanking the excisable fragment. The first origin of replication may be a low-copy number origin of replication, such as *oriS.* The second origin of replication may be an inducible high-copy number origin of replication, such as *oriV.* The high-copy number origin of replication may be under the control of an arabinose promoter. The high-copy number origin of replication may also be regulated by a TrfA encoded by a gene under the control of an arabinose promoter

In another embodiment, the excisable fragment of the vector may comprise at least one type IIS restriction enzyme recognition site. The vector may further comprise two inducible excision-mediating sites flanking the excisable fragment. The excisable fragment may comprise the second origin of replication. In one embodiment, the excisable fragment does not comprise the first origin of replication. In another embodiment, the excisable fragment does not comprise the first or second origin of replication. The vector may also further comprise sequence primer binding sites flanking the excisable fragment. Furthermore, a host cell may comprise the vector.

In one embodiment of the invention, a heterologous nucleic acid may be cloned by providing the heterologous nucleic acid, providing a vector as described herein, and introducing the heterologous nucleic acid into an insertion site of the vector. The vector may have been digested, for example with a TypeII S restriction enzyme. The heterologous nucleic acid may also comprise blunt ends. A double stranded adapter may be provided, which may have a blunt first end and a second end that is complementary to the ends of a digested vector.

In another embodiment, a vector may be produced comprising the cloned heterologous nucleic acid. The heterologous nucleic acid may encode a polypeptide. The heterologous nucleic acid may also be operatively linked to a promoter.

In other embodiments, a library of nucleic acid may be produced by providing a library of heterologous nucleic acids, providing a vector as described herein, and introducing the heterologous nucleic acids into an insertion site of the vector. The vector may have been digested, for example with a TypeII S restriction enzyme. The heterologous nucleic acids may comprise blunt ends. A double stranded adapter may be provided, which may have a blunt first end and a second end that is complementary to the ends of a digested vector.

In yet another embodiment, vectors according to the invention can be used in bacteria-mediated transfer of a gene into cells (bactofection).

### BRIEF DESCRIPTION OF THE DRAWINGS

Figure 1 shows the pBAC-D and pBAC-P vectors constructed from pBAC16.

Figure 2 shows vector constructs for producing a genomic library. Panel A shows pKG15, the progenitor of the vectors. Panel B shows the cassette regions inserted into pKG15. It should be notes that the *lacZ-α* and *PvuIIR* are not drawn to scale.

Figure 3 shows linker classes that may be used to join fragments to the vectors. The 5' overhang of the top strand in the left column is an AarI compatible overhang. The 3' overhang of the top strand in the right column is a BstXI compatible overhang. The 5' overhang of the bottom strand in the left column and the 5' overhang of the top strand in the right column may be any other restriction enzyme.

Figure 4 shows stained tissue culture from Example 6, experiment 5. Sample 1 shows 39% bactofection efficiency (w/ plasmid copy number amplification) and sample 3 shows lack of bactofection efficiency (same plasmid without amplification).

### DETAILED DESCRIPTION OF THE INVENTION

In various embodiments, the present invention is related to vectors that may be used, *inter alia*, for the production of recombinant DNA and proteins, as well as the production of genomic libraries. The present invention is also related to host cells for propagating and maintaining the vectors. Among other beneficial properties, the vectors address the high yield/low stability trade-off that medium- to high-copy number plasmids generally carry and may also reduce basal expression of recombinant proteins.

### 1. Definitions

Before the present compounds, products and compositions and methods are disclosed and described, it is to be understood that the terminology used herein is for the purpose of describing particular embodiments only and is not intended to be limiting. It must be noted that, as used in the specification and the appended claims, the singular forms "a," "an" and "the" include plural referents unless the context clearly dictates otherwise. It must further be noted that the terms "and" and "or" may encompass both conjunctive and disjunctive meaning unless the context clearly dictates otherwise.

The term "base pair" as used herein may refer to the hydrogen bonded nucleotides of, for example, adenine (A) with thymine (T), or of cytosine (C) with guanine (G) in a double-stranded DNA molecule. In RNA, uracil (U) is substituted for thymine. Base pair may also be used as a unit of measure for DNA length.

The term "clone" when used in reference to an insert sequence and a vector may mean ligation of the insert sequence into the vector or its introduction by recombination either homologous, site specific or illegitimate as the case may be. When used in reference to an insert sequence, a vector, and a host cell, the term may mean to make copies of a given insert sequence. The term may also refer to a host cell carrying a cloned insert sequence, or to the cloned insert sequence itself.

The term "compatible" as used herein when referring to two nucleic acid ends may mean that the ends are (i) both blunt or (ii) contain complementary single strand extensions, such as that created after digestion with a restriction endonuclease. At least one of the ends may contain a 5' phosphate group, which may allow ligation of the ends by a double-stranded DNA ligase.

The term "complement," "complementary" or "complementarity" as used herein may mean Watson-Crick or Hoogsteen base pairing between nucleotides or nucleotide analogs of nucleic acid molecules. For example, the sequence 5'-A-G-T-3' is complementary to the sequence 3'-T-C-A-5'. Complementarity may be "partial," in which only some of the nucleotides are matched according to the base pairing rules. Or, there may be "complete" or "total" complementarity between the nucleic acids. The degree of complementarity between nucleic acid strands may have effects on the efficiency and strength of hybridization between nucleic acid strands.

The term "encoding" or "coding" as used herein when referring to a nucleic acid may mean a sequence of nucleotides, which upon transcription into RNA and subsequent translation into protein, would lead to the synthesis of a given protein, peptide or amino acid sequence. Such transcription and translation may actually occur *in vitro* or *in vivo*, or may be strictly theoretical based on the standard genetic code.

The term "free end" when used in reference to a double-stranded nucleic acid may mean a linear nucleic acid with blunt free ends or sticky free ends, or a combination thereof.

The term "gene" as used herein may refer to a nucleic acid (e.g., DNA or RNA) that comprises a nucleic acid sequence encoding a polypeptide or precursor thereto. The polypeptide can be encoded by a full length coding sequence or by any portion of the coding sequence so long as the desired activity or functional properties (e.g., enzymatic activity, ligand binding, signal transduction, antigenicity etc.) of the full-length or fragment are retained. The term also encompasses the sequences located adjacent to the coding region on both the 5' and 3' ends that contribute to the gene being transcribed into a full-length mRNA. The term "gene" encompasses both cDNA and genomic forms of a gene. A genomic form or clone of a gene may contain the coding region interrupted with non-coding sequences termed (e.g., introns).

The terms "library" as used herein may refer to a plurality of vectors each comprising an insert sequence or to a plurality of nucleotide fragments.

The term "nucleic acid" as used herein may mean any nucleic acid containing molecule including, but not limited to, DNA or RNA. The term encompasses sequences that include any base analogs of DNA and RNA including, but not limited to, 4-acetylcytosine, 8-hydroxy-N6-methyladenosine, aziridinylcytosine, pseudoisocytosine, 5-(carboxyhydroxylmethyl)uracil, 5-fluorouracil, 5-bromouracil, 5-carboxymethylaminomethyl-2-thiouracil, 5 carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracils, 5-methoxyaminomethyl-2-thiouracil, β-D-maninosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, N-uracil-5-oxyacetic acid methylester, uracil-5-oxyacetic acid, pseudouracil, queosine, 2-thiocytosine, and 2,6-diaminopurine.

The term "nucleotide" as used herein may refer to a monomeric unit of nucleic acid (e.g. DNA or RNA) consisting of a pentose sugar moiety, a phosphate group, and a nitrogenous heterocyclic base. The base may be linked to the sugar moiety via the glycosidic carbon (1' carbon of the pentose). The combination of base and sugar may be called a nucleoside. When the nucleoside contains a phosphate group bonded to the 3' or 5' position of the pentose, it may be referred to as a nucleotide. A sequence of operatively linked nucleotides may be referred to as a "base sequence" or "nucleotide sequence" or "nucleic acid sequence," and may be represented herein by a formula whose left to right orientation is in the conventional direction of 5'-terminus to 3'-terminus.

The term "operably linked" as used herein may refer to a promoter and downstream polynucleotide, such that productive transcription of the polynucleotide is initiated at the promoter.

The term "restriction" as used herein may refer to the nicking or cleavage of a double-stranded DNA at a recognition site by an endonuclease. The endonuclease may cleave the double-stranded DNA at or near the recognition site.

### 2. Vector

The vector may be a circular or linear nucleic acid molecule capable of replication in a cell and to which an insert sequence can be integrated so as to bring about replication of the insert sequence. Representative examples of vectors include, but are not limited to, plasmids, phagemids, cosmids, yeast artificial chromosomes (YACs) and BACs.

The vector may comprise any of a number of vector elements, such as those described below. The vector may be produced using a combination of *in vitro* and *in vivo* methods such as those described in Sambrook, J., et al., "Molecular Cloning: A Laboratory Manual," which is incorporated herein by reference.

### a. Origin of Replication

The vector may comprise an origin of replication, which may provide for autonomous propagation of the vector in a host cell. Representative examples of origins of replication include, but are not limited to, *oriS,* the Coley origin, and the chromosomal origin of replication from other bacteria such as *Salmonella*, *Yersinia*, *Erwinia* and *Shigella.* The origin of replication may also be *oriV* (requires protein TrfA), fd origin of replication (requires the bacteriophage fd gene II encoded protein), lambda ori (requires lambda O protein) and related origins of replication in phage such as phi 80, phi 21, 434 and 81, the lytic and lysogenic origin of replication for phage, such as P1, the origin of replication from single-stranded Phage M13, and the cis-activated origin of replication of lambda.

The vector or a host cell may comprise the appropriate replication factors for propagation of the vector based on the choice of the origin of replication.

The origin of replication may be a low-copy origin of replication, which may have a partitioning system so that few daughter cells do not comprise the vector as the cells divide. The segregation of the low-copy origin of replication may be correlated with the segregation products of the host chromosome or may be segregated by a parallel mechanism. The low-copy origin of replication may maintain a low number of vectors in a host cell, which may be useful to minimize rearrangements between vector, insert and host chromosome. A low-copy origin of replication may result in 1-5 copies of the vector in a host cell. A representative example of a low-copy origin of replication is *oriS.*

The origin of replication may also be a high-copy origin of replication, which may not have a partitioning system. A high-copy origin of replication may segregate by mass action, which may cause empty cells to be minimized by the low probability in view of the number of vector copies. The high-copy origin of replication may maintain a high number of vectors in a host cell. The high-copy origin of replication may maintain 5-100 copies of the vector in a host cell. The high-copy origin of replication may also maintain more than 100 to more than 1000 copies of the vector in a host cell. A representative example of a high-copy origin of replication is *oriV*, which may require mutant *trfA.* A high-copy origin of replication may be used to increase production of the vector in a host cell.

The vector may comprise more than one origin of replication. For example, the vector may comprise a low-copy origin of replication and a high-copy origin of replication. The vector may be maintained using the low-copy origin of replication, but may be produced in large quantities by inducing expression of a replication factor for the high-copy origin of replication, as described in Hradecna et al. 1998 and Wild et al. 1998, the contents of which are incorporated herein by reference. Such a system may provide "on command" amplification from single copy to high-copy to avoid the metabolic stress and instability of high-copy vectors without sacrificing the benefits.

### b. Markers

The vector may also comprise a selectable or visible marker, which may be a gene, or other DNA fragment that encodes or provides an activity that confers the ability to grow or survive in what would otherwise be a deleterious environment. For example, a selectable marker may confer resistance to an antibiotic or drug upon the cell in which the selectable marker is expressed. An origin of replication may also be used as a selectable marker enabling propagation of a plasmid vector. Selectable or visible markers may be used to maintain and manipulate the cloned DNA. Representative examples of markers include, but are not limited to, Amp^{R}, Cam^{R}, Kan^{R}, Tet^{R}, *lacZ* and the gene encoding green fluorescent protein (GFP). The marker may also be a negatively selected marker, such as a restriction enzyme (e.g., *pvuIIR*), or others described herein.

Antibiotic resistance markers may sometimes entail gene expression that may impose a burden on cell metabolism. Under certain circumstances, it may be desirable to minimize the burden on cell metabolism by using a marker that may be regulated to function when initial clones are being selected but not active during propagation when the metabolic burden may be a problem. For example, Tet resistance expression is regulated so the burden is low when Tet is absent from the medium. If the vector has partitioned regulation, the marker may not be continuously needed to select against cells lacking the vector and thus a marker such as Tet may be used.

### c. Insert Site

The vector may also comprise an insert site, which may be used to clone a nucleic acid. The insert site may be the recognition site of an endonuclease such as a Type I, II or III restriction enzyme, a homing endonuclease, or a nicking enzyme. The insert site may also be a specific site for homologous recombination. The insert site may be present in the vector only at the insert site. In certain circumstances, it may be desirable to remove other insert sites from the vector. For example, when the insert site is the recognition site for a restriction enzyme, it may be desirable to remove other such recognition sites from the chromosome.

Representative examples of Type I restriction enzymes include, but are not limited to, CfrAI, Eco377I, Eco394I, Eco585I, Eco646I, Eco777I, Eco826I, Eco851I, Eco912I, EcoAI, EcoBI, EcoDI, EcoDR2, EcoDR3, EcoDXXI, EcoEI, EcoKI, EcoprrI, EcoR124I, EcoR124II, EcoRD2, EcoRD3, HindI, KpnAI, KpnBI, NgoAV, StyLTIII, StySBLI, StySEAI, StySGI, StySJI, StySKI, StySPI and StySQI. Representative examples of Type III restriction enzymes include, but are not limited to, EcoP15I, EcoPI, HinfIII and StyLTI.

Representative examples of Type II restriction enzymes include, but are not limited to, AarI, AatII, AccI, AceIII, AciI, AclI, AcyI, AflII, AflIII, AgeI, AhaIII, AjuI, AlfI, AloI, AluI, AlwFI, AlwNI, ApaBI, ApaI, ApaLI, ApoI, AscI, AspCNI, AsuI, AsuII, AvaI, AvaII, AvaIII, AvrII, BaeI, Ball, BamHI, BbvCI, BbvI, BbvII, BccI, Bce83I, BcefI, BcgI, BciVI, BelI, BdaI, BetI, BfiI, BglI, BglII, BinI, BmgI, BplI, Bpu10I, BsaAI, BsaBI, BsaXI, BsbI, BscGI, BseMII, BsePI, BseRI, BseSI, BseYI, BsgI, BsiI, BsiYI, BsmAI, BsmI, Bsp1407I, Bsp24I, BspGI, BspHI, BspLU11I, BspMI, BspMII, BspNCI, BsrBI, BsrDI, BsrI, BstEII, BstXI, BtgZI, BtrI, BtsI, Cac8I, CauII, CdiI, Cfr10I, CfrI, CjeI, CjeNII, CjePI, ClaI, CspCI, CstMI, CviJI, CviRI, DdeI, DpnI, DraII, DraIII, DrdI, DrdII, DsaI, Eam1105I, EciI, Eco31I, Eco47III, Eco57I, Eco57MI, EcoNI, EcoRI, EcoRII, EcoRV, Esp3I, EspI, FalI, FauI, FinI, Fnu4HI, FnuDII, FokI, FseI, FspAI, GdiII, GsuI, Hael, HaeII, HaeIII, HaeIV, HgaI, HgiAI, HgiCI, HgiEII, HgiJII, HhaI, Hin4I, Hin4II, HindII, HindIII, HinfI, HpaI, HpaII, HphI, Hpy178III, Hpyl88I, Hpy99I, KpnI, Ksp632I, MaeI, MaeII, MaeIII, MboI, MboII, McrI, MfeI, MjaIV, MluI, Mmel, MnlI, MseI, MslI, MstI, MwoI, NaeI, NarI, NcoI, NdeI, NheI, NlaIII, NlaIV, NotI, NruI, NspBII, NspI, OliI, PacI, PasI, Pfl1108I, PflMI, PfoI, PleI, PmaCI, PmeI, PpiI, PpuMI, PshAI, PsiI, PspXI, PsrI, PstI, PvuI, PvuII, RleAI, RsaI, RsrII, SacI, SacII, SalI, SanDI, SapI, SauI, ScaI, ScrFI, SduI, SecI, SexAI, SfaNI, SfeI, SfiI, SgfI, SgrAI, SgrDI, SimI, SmaI, SmlI, SnaBI, SnaI, SpeI, SphI, SplI, SrfI, Sse232I, Sse8387I, Sse8647I, SsmI, SspI, Sth132I, StuI, StyI, SwaI, TaqI, TaqII, TatI, TauI, TfiI, TseI, TsoI, Tsp45I, Tsp4CI, TspDTI, TspEI, TspGWI, TspRI, TssI, TstI, TsuI, Tth111I, Tth111II, UbaF10I, UbaF9I, UbaPI, VspI, XbaI, XcmI, XhoI, XhoII, XmaIII and XmnI.

Representative examples of homing endonucleases include, but are not limited to, F-SceI, F-SceII, F-SuvI, F-TevI, F-TevII, F-TflI, F-TflII, F-TflIV (also known as HegA), H-DreI, I-AmaI, I-AniI, I-BasI, I-Bmol, I-Ceul, I-CeuAIIP, I-ChuI, I-CmoeI, I-CpaI, I-CpaII, I-CreI, I-CrepsbIP, I-CrepsbIIP, I-CrepsbIIIP, I-CrepsbIVP, I-CsmI, I-CvuI, I-CvuAIP, I-DdiI, I-DirI, I-DmoI, I-HmuI, I-HmuII, I-HspNIP, I-LlaI, I-MsoI, I-NaaI, I-NanI, I-NclIP, I-NgrIP, I-NitI, I-NjaI, I-Nsp236IP, I-PakI, I-PbolP, I-PculP, I-PcuAI, I-PcuVI, I-PgrIP, I-PobIP, I-PogI, I-PorI, I-PorIIP, I-PpbIP, I-PpoI, I-ScaI, I-SceI, I-SceII, I-SceIII, I-SceIV, I-SceV, I-SceVI, I-SceVII, I-SneIP, I-SpomI, I-SquIP, I-Ssp6803I, I-SthPhiJP, I-SthPhiST3P, I-SthPhiS3bP, I-TevI, I-TevII, I-TevIII, I-Tsp061I, I-TwoI, I-UarHGPA1P, I-VinIP, I-ZbiIP, PI-MgaI, PI-MtuI, PI-MtuHIP, PI-MtuHIIP, PI-PabI, PI-PabII, PI-PfuI, PI-PfuII, PI-PkoI, PI-PkoII, PI-PspI, PI-Rma43812IP, PI-ScaI, PI-SceI, PI-TfuI, PI-TfuII, PI-ThyI, PI-TliI, PI-TliII and PI-ZbaI.

Other possible types of endonucleases are enzymes characterized by the complexity of their recognition sites. A representative example of such an enzyme is FseI, as described in U.S. Patent No. 5,543,308, which is incorporated herein by reference.

The vector may comprise a plurality of insert sites and the insert sites may be clustered as part of a multiple cloning site. The vector may also comprise more than one multiple cloning sites, which may be identical.

### d. Stuffer Fragment

The vector may also comprise a stuffer fragment, which may be defined at each end by a recognition site for an endonuclease. The stuffer fragment may comprise an origin of replication. The stuffer fragment may also comprise a marker. The stuffer fragment may also comprise an insert site.

The recognition sites defining the ends of the stuffer fragment may be restriction sites for a class-IIS or similar enzyme. Such recognition sites may be used to produce non-palindromic and non-complementary overhangs at the two ends of the linearized vector. For example, digestion with AarI may be used to produce the following overhangs of the resulting vector fragment:

5'pGAAGNNNNN----------------------------------------------NNNNN3'

          NNNNN-------------------------------------------NNNNNGAAGp5'

The two ends of the linear vector fragment may not ligate to one another to re-close the vector because they are not complementary. An adapter may be used to ligate an insert to the linearized vector. The adapter may comprise two partially complementary strands of DNA that are annealed to one another to produce a duplex DNA molecule with an overhang complementary to the vector at a one end. The other end of the adapter may be a blunt end. The adapter may lack a phosphate at both ends. The adapter may also contain sites for restriction enzymes. A representative example of an adapter for the linearized vector shown above is the following, which also has recognition sites for NotI (GC▼GGCCGC) and AscI (GG▼CGCGCC):

5'CTTCGGCGCGCCGCGGCCGC3'

3'    CCGCGCGGCGCCGGCG5'

The adapter may not ligate to itself because it does not have a 5' phosphate on either strand and because the overhang is not complementary to itself. Upon ligation of the target fragments, vector and adapter, only the following reactions may take place: (a) the joining of the blunt ends of the adapter to blunt phosphorylated ends of target fragments, (b) annealing followed by joining of adapter overhangs to the complementary overhangs of the vector, and rarely (c) joining of target ends to produce concatenated targets. The vastly preferred reaction product may be a circle containing one vector joined to one target via a single adapter at each end. This molecule may be transformed into host cells to produce a clone. Side reaction products (c) may not be distinguished and may not be clonable because of their nearly double or even larger size.

The blunt ends of the adapter may be adjusted to be complimentary to the target fragments. For example, the target fragments may be randomly sheared DNA which may be made blunt by repair. The blunt DNA may be modified by non-template mediated addition of a single A nucleotide to each end of the linear target DNA by Taq polymerase. In such a case, the above adapter may be modified as follows:

5'CTTCGGCGCGCCGCGGCCGCT3'
3'    CCGCGCGGCGCCGGCG 5'

The addition of the single A nucleotide to the target DNA may prevent formation of linear concatemers of DNA and may increase the efficiency of ligation of linkers to the target fragments. Additional methods for achieving identical non-complimentary overhangs include polynucleotide addition by a terminal transferase and the use of proofreading activities of a DNA polymerase in the presence of three or fewer nucleotide triphosphates to a unique position defined by a base not in the mixture.

The stuffer fragment may be removed by physical means after linearization of the vector. The stuffer fragment may also contain a selectable or assayable marker, which may be used to select or identify host cells comprising a vector with a nucleic acid cloned into the insertion site. The selectable marker may encode *LacZ*, which allows identification of clone inserts by color. The selectable marker may also be a form of negative counterselection. For example, the stuffer fragment may comprise a negatively selectable marker for sucrose sensitivity, such as that encoded by *sacB* of *B. subtilis.*

The stuffer fragment may also comprise the gene for the "stuck open" mutant of the osmoregulator MscL of E. coli under the control of the arabinose promoter, lac promoter or another promoter. The *mscL* gene encodes a transmembrane channel for water, which is normally opened or closed to maintain homeostatic osmotic pressure in the cell. The "stuck-open" mutant channel is always open so its expression produces holes in the membrane that cannot be closed, and therefore, the cell dies, which provides a negative selection. Conditions for propagating the uncut vector are chosen under which the toxic product is not produced (e.g., repression with glucose) and conditions for selection and propagation of the clones are chosen under which the toxin is expressed (e.g., in the presence of arabinose).

The stuffer fragment may also comprise a sequence encoding a restriction enzyme, for example, PvuII. The restriction enzyme may be specific for a restriction site, such as a restriction site listed herein. The vector may be maintained in a host cell that expresses a methyltransferase gene specific for the restriction site recognized by the restriction enzyme.

The negative selection may also be the toxin (Doc) antitoxin (Phd) system from bacteriophage P1. The vector may be propagated in a host expressing the antitoxin, Phd. Recombinant clones may be propagated in another host which does not express Phd. After isolation of the vector DNA, it may be cut with a cloning enzyme and purified away from the stuffer fragment. The target fragment may be inserted using one of the above methods and the ligation product introduced into the propagation host. Vectors retaining the stuffer fragment will express the toxin Doc, killing the cell. The desired clones containing inserts but no stuffer fragment are propagated normally. The negative counter selection may also be a method as described in U.S. Patent No, 6,291,245, which is incorporated herein by reference.

### e. Transcription Terminators

The vector may also comprise a transcriptional terminator, which may be located in the vector such that transcription initiated from a promoter outside the stuffer fragment does not extend transcription into the stuffer fragment. The vector may comprise a transcriptional terminator on each side of the stuffer fragment. This may allow improved cloning of nucleic acid sequences encoding potentially toxic products.

The transcription termination sequence may comprise a GC-rich region that has a twofold symmetry followed by an AT-rich sequence. A variety of transcription termination sequences may be used including, but not limited to, the T7, T_{INT}, T_{L1}, T_{L2}, T_{L3}, T_{R1}, R_{R2}, T_{6S}, termination signals derived from the bacteriophage lambda, termination signals derived from bacterial genes such as the *trp* gene cluster of *E*. *coli,* and ribosomal terminators such as T_{rrnB}.

### f. Sequencing Primers

The vector may also comprise a sequencing primer binding site, which may be used for sequencing nucleic acids cloned into the insert sequence. The vector may comprise a sequencing primer binding site on each side of the stuffer fragment. A variety of sequencing primer binding sites may be used including, but not limited to, -47 and -48.

### g. Excision

The vector may also comprise excision sites, which may flank the insert site and thereby define an excisable fragment. In the presence of a suitable signal and with the appropriate factors, excision sites may be activated to recombine with one another and thereby excise the excisable fragment, and a nucleic acid within the insert site, to produce a circular plasmid.

The excisable fragment may comprise an origin of replication, which may be used to replicate the excised fragment. The excisable fragment may also comprise a marker, which may be used to select for the excised fragment. The excisable fragment may also not have an origin of replication or a marker, for example, in those cases where it is desired to have a minimum amount of nucleic acid sequence other than a cloned insert.

The excision sites may be compatible with systems including, but not limited to, lambda or other phage integrases with or without xis (e.g., attP and attB sites), cre-lox, flip recombinase (e.g., FRT elements) and lambda minicircles. The excision system may be inducible, as described in U.S. Patent No. 5,874,259, which is incorporated herein by reference.

### 3. Host cells

The host cell may be any cell that can be transformed with the vector. Representative examples of host cells include, but are not limited to, *E*. *coli* strains such as K-12 or B. The K-12 strain may be MG1655. The *E*. *coli* strain may lack the F or F' factor (e.g. DH10B). The host cell may also be a reduced genome bacteria, as described in WO 2003/070880, the contents of which are incorporated herein by reference. The reduced genome bacteria may be modified to lack insertion sequences and recognition sites of certain endonucleases.

The host cell may also comprise factors that lead to the replication of the origin of replication, and these factors may be inducible. For example, the host cell may comprise the *trfA* gene, the product of which may allow maintenance of a high-copy number origin of replication such as *oriV.* Expression of the *trfA* gene may be under control of an inducible promoter, such as the arabinose-controlled promoter *P_{BAD}* promoter.

### 4. Cloning

The vector may be a cloning vector, which may be used to clone a nucleic acid fragment. The vector may be cut at the insert size with an endonuclease compatible with the ends of the nucleic acid fragment to be cloned. The nucleic acid fragment may then be ligated into the vector. Ligation may occur in a single stage or in multiple stages. Adapters may be used to ligate the nucleic acid fragment to the insert site of the vector. The nucleic acid fragment may also be introduced into the cloning vector by homologous, site specific or bridging recombination either *in vitro* or *in vivo.*

The nucleic acid fragment may be any nucleic acid sequence that is capable of being placed in a vector. Representative examples include, but are not limited to, random DNA libraries, genomic libraries and known nucleic acid sequences. A particular nucleic acid fragment may refer to a pool or a member of a pool of identical nucleic acid molecules, a pool or a member of a pool of non-identical nucleic acid molecules, or a specific individual nucleic acid molecule.

The nucleic acid fragment may be randomly sheared DNA, which may have blunt ends. The randomly sheared DNA may be size-selected using, for example, pulsed field gel electrophoresis. The ends of the randomly sheared DNA may be repaired to produce blunt phosphorylated ends. One or more nucleotides may be added to an end of a strand by non-template mediate addition, such as the addition of a 3' adenosine nucleotide by Taq polymerase. The randomly sheared DNA may be ligated to the vector using an adapter, which may be in excess to the nucleic acid fragment.

### 5. Protein Expression

The vector may be an expression vector, which may comprise appropriate nucleic acid sequences necessary for expression of an operably linked coding sequence (e.g. an insert sequence that codes for a product) in a particular host organism. Nucleic acid sequences necessary for expression include, but are not limited to, a promoter, an operator, and a ribosome binding site.

The promoter may be an inducible promoter, which may function in the host cell by responding to an inducing agent to promote transcription of an operably linked downstream polynucleotide. The promoter may be inactive prior to induction resulting in insignificant or undetectable levels of product as measured by conventional detection methods in the non-induced state, which may be desired if expression of a particular polypeptide is deleterious to the host cell. Representative examples of promoters include, but are not limited to, AraC/P_{araBAD} (activator-promoter), which can be induced by arabinose, the TetR/P_{LtetO} (repressor-promoter), which can be induced by anhydrotetracycline, P_{tac}, and the T5, T7 and rhamnose promoters. When the promoter is that of phage T7, the host cell may express T7 polymerase under control of another inducible promoter, such as the rhamnose promoter.

The vector may comprise a coding region for an affinity tag, which may be used in purifying expressed proteins. The coding region may allow expression of the affinity tag at the amino- or carboxy-terminus of the expressed protein. The coding region may also comprise a protease cleavage site, which may be used to cleave the affinity tag after purification of the expressed protein. Representative examples of affinity tags include, but are not limited to, (His)ₙ, maltose binding protein, thioredoxin, glutathione S-transferase and NusA.

### 6. Bactofection.

In one embodiment, vectors asdescribed herein can be used to deliver nucleic acid, for example a gene, or endogenous, exogenous or heterologous expressible DNA or RNA encoding or comprising therapeutic or prophylactic agents, into an animal cell (bactofection). Bacteria used in these methods are referred to as bacterial vectors or bactofection vectors. Therapeutic or prophylactic agents encoded by nucleic acid may include synthetic genes, immunoregulatory agents, antigens, for example, antigens associated with pathogenic organisms or tumors, DNAs, antisense RNAs, catalytic RNAs, proteins, peptides, antibodies, cytokines small molecules or other useful therapeutic or prophylactic molecules.

In one embodiment, the invention comprises a method of inducing and expressing nucleic acid or gene in an animal cell, the method comprising: (a) providing a vector comprising an insertion site, a first and second origin of replication and a pair of transcriptional terminators; (b) introducing the nucleic acid or gene into the insertion site of the vector; (c) transforming at least one invasive bacterium with the vector to form at least one transformed bacterium; and (d) infecting the animal cell with said transformed bacterium. In one embodiment, the nucleic acid or gene is expressed at detectable levels. In another embodiment, the animal cells are cultured *in vitro*

An "invasive bacterium" herein is a bacterium naturally capable of entering the cytoplasm or nucleus of animal cells, as well as bacterium that are genetically engineered to enter the cytoplasm or nucleus of animal cells.

In a related embodiment, the first origin of replication is a low-copy number origin of replication. In another embodiment, the low-copy number origin of replication is *oriS._*In yet another embodiment, the second origin of replication is an inducible high-copy number origin of replication. In still another embodiment, the high-copy number origin of replication is *oriV.* In one embodiment, the high-copy number origin of replication is under the control of an arabinose promoter. In another embodiment the high-copy number origin of replication is regulated by a TrfA encoded by a gene under the control of an arabinose promoter.

In another embodiment, the vector further comprises two inducible excision-mediating sites flanking the excisable fragment. In another embodiment, the excisable fragment comprises the second origin of replication. Alternately, the excisable fragment does not comprise the first origin of replication. In a related embodiment, the excisable fragment does not comprise the first or second origin of replication encoding or comprising therapeutic or prophylactic agent.

In one embodiment, the nucleic acid or gene encodes a therapeutic or prophylactic agent. In another embodiment, the nucleic acid comprises DNA or RNA encoding a member selected from the group consisting of a therapeutic protein, a small molecule, an immunoregulatory molecule, antisense RNA, and catalytic RNA. In another embodiment, the encoded member is expressed at least at a detectable level.

In another embodiment, the mammalian cells are selected from the group consisting of human, bovine, ovine, porcine, feline, buffalo, canine, goat, equine, donkey, deer, and primate cells.

In another embodiment, the invasive bacteria are selected from the group consisting of *Shigella spp, Listeria spp*., *Rickettsia spp and enteroinvasive Escherichia coli.* In another embodiment, the invasive bacteria comprise *Yersinia spp*., *Escherichia spp*., *Klebsiella spp*., *Bordetella spp*., *Neisseria spp., Aeromonas spp*., *Franciesella spp*., *Corynebacterium spp*., *Citrobacter spp*., *Chlamydia spp., Hemophilus spp., Brucella spp*., *Mycobacterium spp*., *Legionella spp., Rhodococcus spp*., *Pseudomonas Spp*., *Helicobacter spp*., *Salmonella spp*., *Vibrio spp*., *Bacillus spp*., *Leishmania spp. or Erysipelothrix spp. which have been genetically engineered to mimic the invasion properties of Shigella spp*., *Listeria spp*., *Rickettsia spp*., *or enteroinvasive E. coli spp.* In another embodiment, the bacteria have a reduced genome. In yet another embodiment, the bacteria are attenuated. In still another embodiment, the bacterial strain is MDS42.

In another embodiment, the animal cells are derived from or present in multicellular organisms. The cells may be present in intact animal, a primary cell culture, explant culture or a transformed cell line.

In another embodiment, the invention is directed to therapeutic or prophylactic methods in which the bacterial vectors of the present invention are administered to animals, preferably humans, for the purpose of treating or preventing diseases.

In another embodiment, vectors and plasmids of the invention provide improved bactofection efficiency, for example exhibiting greater than about 1%, greater than about 5%, greater than about 10%, greater than about 20%, greater than about 30%, greater than about 40%, greater than about 50%, greater than about 60%, greater than about 70%, greater than about 80% or greater than about 90% bactofection.

### EXAMPLES

These and other embodiments of the invention are exemplified in the following nonlimiting examples.

### Example 1

### Amplifiable Vectors For DNA And Protein Production

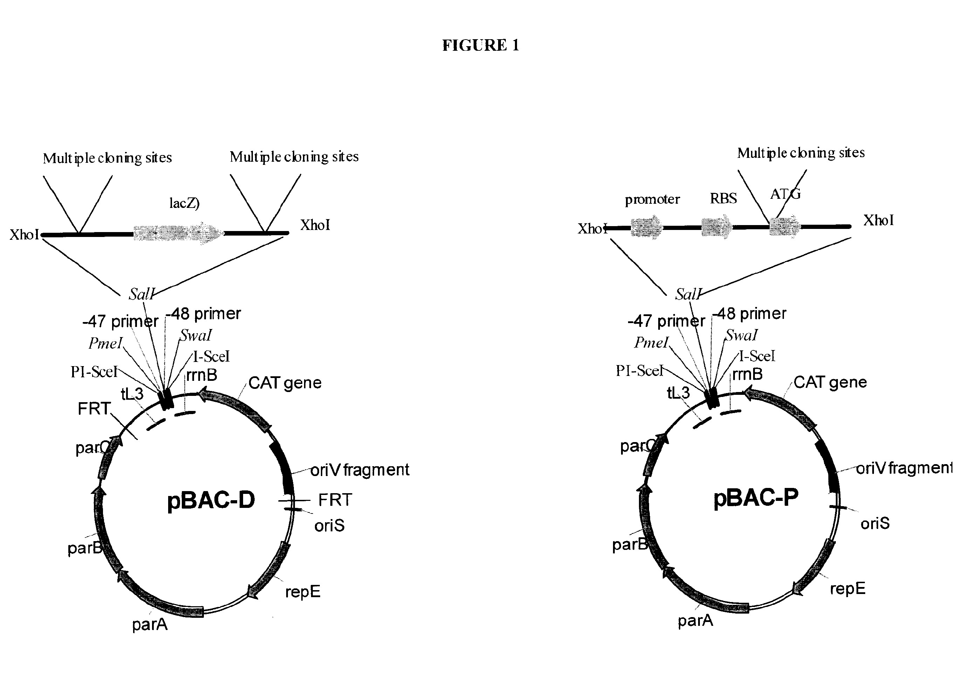

The vectors of Figure 1 feature "on command" amplification from single copy to high-copy to avoid the metabolic stress and instability of high-copy vectors without sacrificing the benefits. The vectors were derived from plasmid pBAC16. The plasmid may be maintained as a single copy by the *oriS* origin of replication and during cell division, a single copy of the plasmid may be faithfully transferred to a daughter cell by partitioning functions encoded by the *par* genes. This origin may stably maintain inserts greater than 200 kb, allowing the introduction of whole metabolic pathways.

It should be noted that *oriS* may allow a selection-based drug to be omitted in the maintenance of the plasmid. Omitting drug selection may reduce stress on the host cells as well as minimize production costs. It should also be noted that the CAT gene and *OriV* fragment are depicted as within the two FRT sites, but either or both could be located outside of the two FRT sites depending on whether it is desirable for either or both to be present on an excision product. It should also be noted that *parA*, *parB* and *parC*, which are involved in the partitioning of *OriV*, and *repE*, which is involved in the replication of the F' plasmid, are not necessary and if desired may be located on the vector or may be provided by the host cell.

The plasmid also contains a second origin of replication (*oriV*) which may be activated to high-copy number by expression of the *trfA* gene product. The *trfA* gene product may initiate replication at *oriV* and produces up to 100 copies per cell. The *oriV* origin may work with inserts of greater than 150 kb with no deleterious effects. The combination of two origin of replications allows for stable replication of the plasmid at low-copy as well as amplification to high-copy.

Amplifiable BAC vectors, such as those described in U.S. Patent Nos. 5,874,259, 6,472,177 and 6,864,087, which are incorporated herein by reference, have the problem that the cloning site is in a region of convergent transcription between the *parC* gene and the *cat* gene. Read through transcription into the cloned DNA often causes problems with instability. The vectors pBAC-P and pBAC-D have transcription terminators flanking the SalI cloning site, which may prevent transcription read through into the target DNA. Sequencing primer binding sites (-47 and -48 primers), and rare-cutting enzyme sites (PmeI, PI-SceI, SwaI and I-SceI) also flank the SalI cloning site for convenience in cloning.

The unique SalI site of pBAC16 may be used to modify the vector for additional applications and products. Inserts may be cloned as SalI fragments, in which case the SalI site is recreated or as XhoI fragments which have compatible overhangs but do not recreate the SalI or XhoI site. Cassettes with multiple cloning sites, color screen or special optimizations for individual experiments may be easily designed and cloned into the pBAC16 vector.

Vector pBAC-D, which may be used for cloning, was produced by adding a cloning cassette to pBAC16 at the SalI site. Traditional cloning cassettes take advantage of cloning into the alpha fragment of the *lacZ* gene, causing inactivation of *lacZ* which can be easily assayed as blue/white colonies on X-gal plates. The disadvantage of this system is that the *lac* promoter is still present and can direct transcription into the cloned fragment, causing problems if expression from the insert is deleterious. To take advantage of the transcriptional terminators in pBAC16, the cloning cassette had a restriction site on both sides of *lacZ*, which allows complete removal of the *lacZ* promoter and alpha fragment. A DNA may thus be cloned between two transcriptional terminators with no promoters.

Vector pBAC-P, which may be used for protein expression, was produced by adding an expression cassette at the SalI site of pBAC16. The cassette contained the T7 promoter, a ribosome binding site and multiple cloning sites. For protein expression, especially involving toxic products, a low basal level of expression may be very important for clone stability and robust growth. The amplifiable vector in single copy may provide two dimensions for achieving a low basal level: (i) expression systems with a low basal level in ordinary multi-copy vectors may have even lower basal level at single copy; and (ii) read through transcription into the vector may be strictly limited by the flanking transcription terminators. To achieve high but controllable expression, both the promoter and the copy number may be induced. Since induction of replication combines with induction of transcription, a very wide dynamic range may be achieved

### Example 2

### Independently Controlling Induction And Copy Number

An advantage of the pBAC-P vector is that it is possible to regulate copy number and transcription independently to control protein expression. One method of induction is the use of diauxic shift, in which host cells are grown in a limiting amount of glucose with an additional sugar such as lactose, maltose, rhamnose or arabinose in the medium whose metabolism is catabolite repressed. When glucose is consumed, catabolite repression is alleviated and promoters under control of the supplied secondary sugar may be activated. In some protocols, glycerol is also added to the medium to provide a source of energy after the diauxic shift. Although it is not catabolite repressed, glycerol may not be used if glucose is available. Auxotrophic mutations are often introduced intentionally into the catabolic pathway of the inducing sugar to prevent its metabolism to stabilize the level of induction. Glycerol may be needed in these cases to provide a source of energy.

The diauxic shift method may be self regulating. No matter what the growth rate of the cells, they may run out of glucose and induce at the same point in the growth curve. There is no need to monitor growth and then induce manually. This is especially attractive in high throughput applications in microtiter plates where the innoculum and growth rate of different isolates varies. Diauxic shift may involve no expensive chemicals like IPTG, no complex equipment as required for temperature shift and no metabolic assault on the cells as in phosphate starvation. The method is inexpensive, reproducible and convenient.

We constructed reduced genome bacteria strains with *trfA* under the control of arabinose, and the T7 polymerase gene under rhamnose control. We have validated that diauxic shifts work well in both cases individually. Arabinose-controlled *trfA* and rhamnose-controlled T7 polymerase are combined in MDS43. In this construct, the catabolic genes for arabinose and rhamnose are replaced with *trfA* and T7 polymerase, respectively, to prevent catabolic reduction of the inducer concentration. Expression from the arabinose promoter may suffer from the "all or none" expression effect where some cells in a population are fully expressing while others are not expressing. Constitutive expression of the arabinose transporter (*araE*) may alleviate this problem. The MDS cell line is constructed to have constitutive expression of *araE.*

Using this host strain and pBAC-P, diauxic shift may be used with arabinose to get amplification without expression. With rhamnose, expression may be induced without amplifying. Using a combination of the two sugars, both amplification and expression may be induced.

### Example 3

### Vectors For Construction Of A Genomic Library

A genomic library is produced by exchanging or replacing a stuffer fragment between two restriction sites of a vector with randomly sheared DNA. The stuffer fragment may contain a selectable or assayable marker. The two class IIS restriction sites or analogous sites which have defined recognition sequences but have undefined bases at the cut site are used, which may allow the creation of identical, non-compatible overhangs. Linkers with compatible overhangs are ligated to the randomly sheared genomic DNA and then ligated to the vector.

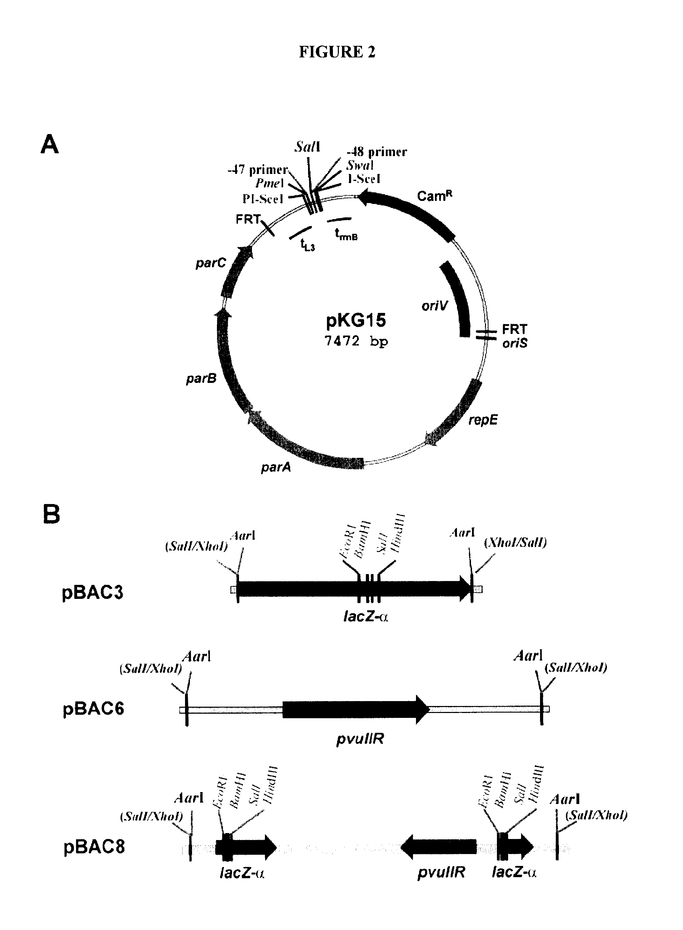

The progenitor of the vector is pKG15, as shown in Figure 2A. Plasmid pKG15 contains five unique features: (1) the chloramphenicol acetyl transferase-encoding gene (*cat*) was modified to remove the internal EcoRI site and to reduce the size of the vector by approximately 600 bp near *oriV*, as compared to the amplifiable BAC vectors described in U.S. Patent Nos. 5,874,259, 6,472,177 and 6,864,087 (2) transcriptional terminator *t_{L3}* and ribosomal terminator *t_{rrnB}* were inserted into the vector, which may reduce read through and may eliminate instabilities and toxicity caused by transcription into or out of the insert; (3) rare restriction sites were inserted flanking the cloning site, which may facilitate clean excision of the cloned DNA fragment and mapping: PmeI (8 bp recognition) and PI-SceI (36 bp) on one side and SwaI (8 bp) and I-SceI (18 bp) on the other; (4) -47 and -48 sequencing primer annealing sites; and (5) a unique SalI restriction site between the sequence primer annealing sites into which various cassettes may be cloned.

It should be noted that *CAT^{R}* and *oriV* are depicted as within the two FRT sites, but either or both could be located outside of the two FRT sites depending on whether it is desirable for either or both to be present on an excision product. It should also be noted that *parA*, *parB* and *parC*, which are involved in the partitioning of *oriV*, and *repE*, which is involved in the replication of the F' plasmid, are not necessary and if desired may be located on the vector or may be provided by the host cell.

Three of the cassettes are shown in Figure 2B. These fragments have XhoI sites on the ends which may be used to clone the fragments into the unique SalI site in pKG15. The type IIS restriction site (AarI) is located interior to the XhoI sites, which may be used to produce a 4 bp 5'-overhang. The cassette of pBAC3 has the *lacZα* complementing fragment between the AarI sites. This vector may allow for two modes of library construction: linker-mediated cloning using the AarI sites, and traditional cloning by insertion into the *lacZ* fragment. In the latter, the clone is placed downstream of the *lac* promoter which may cause problems with toxic clones. The linker-mediated cloning has the benefit of insertion into a transcription free environment.

The cassette of pBAC6 incorporates the gene encoding PvuII restriction endonuclease (*pvuIIR*), which may be used as a negative selectable marker, and may be maintained in a host expressing the PvuII methyltransferase encoding gene (*pvu*II*M*)*.* This vector may be used for linker-mediated cloning. The cassette of pBAC8 combines both the *lacZ* and *pvuIIR* markers. This vector may be used for replacement cloning with EcoRI, BamHI, SalI or HindIII. The *pvuIIR* may be used to select against uncut vectors while *lacZ* may provide blue/white selection.

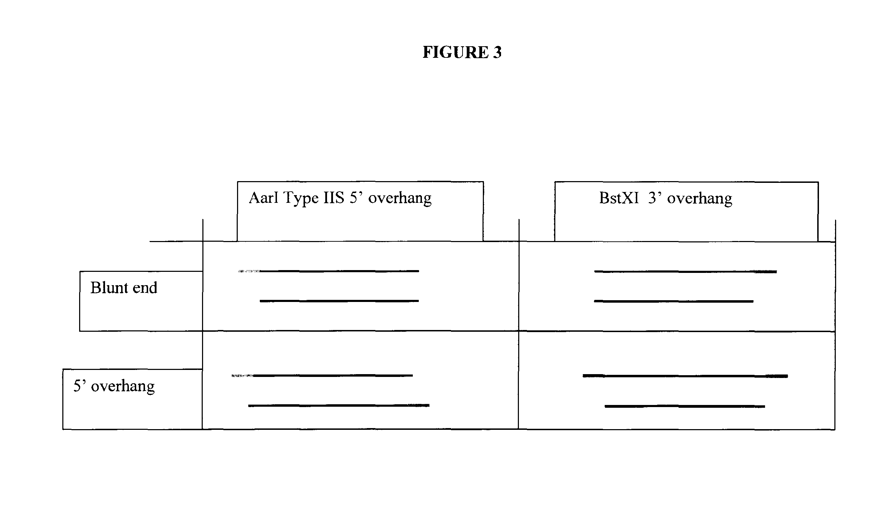

It is possible to change the specific recognition site and/or overhang because the type IIS recognition and restriction site is located within the stuffer fragment. The vector pDW11 was also constructed, which is analogous to pBAC3 except BstXI is used in place of AarI. BstXI leaves a 3'-overhang whereas AarI produces 5'-overhangs. A series of linkers were produced as depicted in Figure 3. The internal double-stranded region contains sites for 8 bp recognition restriction enzymes, NotI and either AscI or PacI. The use of linkers allows the addition of unique enzyme sites flanking the cloned DNA, and may be designed to have a compatible vector-overhang when cleaved with the same enzyme. The linker may either be blunt-end ligated to randomly sheared genomic DNA, or have an overhang compatible with any restriction enzyme.

A 1.5 kb fragment was digested with HindIII and ligated to HindIII compatible linkers, or blunted with T4 polymerase to simulate the cloning of randomly sheared genomic DNA. Both phosphorylated and unphosphorylated linkers with either AarI or BstXI overhangs were ligated to the fragment and then into the appropriately digested vector. The phosphorylated linkers produced more clones than the unphosphorylated linkers, and the BstXI linker/vector combination performed better than AarI. It is not clear if the difference between the two enzymes is due to purity of the restriction enzymes or the fact that one produces 5'-overhangs and the other 3'-overhangs. The blunt-end ligation was comparable to the HindIII overhang in cloning efficiency.

It is possible to design overlapping type IIS restriction sites, because type IIS restriction enzymes bind at one site and cut at a distal site. We designed one vector using BstXI and AarI, with one end of the insert shown below.

XhoI BstXI AarI
5' NNNC+TCGA GCCAN=NNNN^NTGGGCAGGTG
3' NNNG AGCT+CGGTN^NNNN=NACCCGTCCAC

The BstXI and AarI sequence is present on both ends of the stuffer fragment and was cloned into the SalI site of pKG15 using the compatible XhoI sites (+). The 4 bp overhang sequence (shown as NNNN) can be chosen to be any desired sequence. Digestion with BstXI (^) produces 3' overhang and AarI (=) produces 5' overhangs from the same sequence. By combining different restriction enzyme recognition sites, we are able to determine the effect of the overhang sequence, directionality, and enzyme. This same strategy may be extended to type IIE restriction enzymes (e.g., PpiI, Fall, and PsrI) that produce longer overhang sequences to determine the effect of overhang length.

### Example 4

### Vector-Host System

Cloning vector pBAC8 and *E. coli* host MDS42 *trfA* have several unique properties. pBAC8 contains two origins of replication: *oriS*, which assures a stable, single-copy state, and *oriV* that upon arabinose induction of the host *trfA* gene allows BAC8 amplification, facilitating various DNA manipulations, including sequencing. MDS42 *trfA*, was constructed from MDS42 which lacks all transposable elements, to avoid contamination of cloned DNA with these undesirable sequences.

Two modes can be employed to clone inserts in such way that they are flanked by terminators which prevent transcription into or out of cloned DNA. Furthermore, insert-containing clones are selected by both or either a white-blue *lacZ* color screen and a negative counter-selection for uncut vector, based on the lethality of a retained restriction enzyme (REase). Only the propagation of the REase-producing vector, but not of the clones, required a host expressing the cognate methyltransferase.

In the first mode of cloning, DNA fragments are ligated into a multiple cloning site (MCS) in the α fragment of the *lacZ* gene. Such cloning also removes the counter-selected marker (REase-encoding gene). This selects for clones with insert.

In the second mode of cloning, the vector is cut with a type IIs REase at sites (flanking the counter-selected marker and the MCS within *lacZ*) to produce identical, and thus incompatible overhangs. Linkers are then used to make the vector and insert overhangs compatible for efficient ligation. A multitude of linkers was designed for introduction of suitable REase sites at both ends of the insert. This mode produces clones that are completely free of any vector-derived promoters.

We have tested pBAC8 for construction of genomic libraries from chicken DNA that was partially digested with the appropriate restriction endonuclease. The inserts were ligated using linearized vector and the appropriate adapter. Ligation controls were also performed. The efficiency of ligation was analyzed by determining the number of transformants per 0.2 ml of the transformation reaction, with the results as follows:

| # | Ligation | Host cell | Colonies |
|---|---|---|---|
| 1 | pBAC8 only | EPI300 | 0 |
| 2 | pBAC8 only | MDS42trfArecA | 0 |
| 3 | pBAC8 + linker only | EPI300 | 0 |
| 4 | pBAC8 + linker only | MDS42trfArecA | 0 |
| 5 | pBAC8 + linker + insert | EPI300 | 160 |
| 6 | pBAC8 + linker + insert | MDS42trfArecA | 10 |

As expected, controls (#1-#4) produced no transformants. Transformants from #5 and #6 were tested for the presence of large inserts. For #5, 13 of the 16 clones tested had large inserts. For #6, 6 of the 10 clones tested had large inserts. The inserts of the clones ranged from 40 to 70 kb. This shows that the cloning in both hosts resulted in highly efficient cloning of large genomic inserts.

### Example 5

### Construction of Genomic Libraries

A vector similar to pBAC8 was constructed incorporating a BstXI site. The SalI-ApaLI fragment was deleted from pBAC8 to produce pJW680. The HindIII fragment from pBAC8 was then cloned into pJW680 to yield pJW681. The BstXI site of pBAC8 is removed.

Adapters were designed with one end compatible with BstXI. Libraries are constructed using chicken DNA partially digested with the appropriate restriction endonuclease, which was ligated using linearized vector and the adapter. Ligation controls are also performed.

### Example 6

### Vectors for use in Bactofection

A vector similar to pKG15 was constructed containing a CMV promoter controlling expression of a lacZ gene containing intron 2 from the human beta globin gene and a Yersinia pseudotuberculosis invasion gene under its native promoter to produce plasmid pYinv4. The plasmid was transformed into strain MDS42 containing the trfA gene under control of the chromosomal promoter for Ara_{BAD} to allow for plasmid copy number induction.

The MDS42trfA strain containing pYinv4 was grown in 0.02% glucose, and 0.2% arabinose to induce a diauxic shift causing induction of trfA expression from the arabinose promoter and amplifying plasmid copy number. The copy number induced cells were used either fresh or after freezing in 15% glycerol for bactofection of mammalian cells.

The live bacterial cells were added to mammalian HeLa cell cultures and allowed to infect for 2 hours. The HeLa cells were then washed with antibiotics, supplied with fresh media and grown for an additional 18-24 hours. The HeLa cells were then fixed and stained for betagalactosidase activity.

The efficiency of bactofection observed is presented below:

| | Growth conditions: | Percent bactofection |
|---|---|---|
| Exp5: | 1 pYinv4 amplified MDS42RR trfA with arabinose | 39% |
| | 2 pYinv4 amplified MDS42RR trfA with arabinose | 36% |
| | 3 pYinv4 unamplified MDS42RR trfA | 0 |
| | 4 pYinv4 unamplified MDS42RR trfA | 0 |
| | 5 no plasmid MDS42RR trfA amplified trfA | 0 |
| | 6 no bacterial culture | 0 |

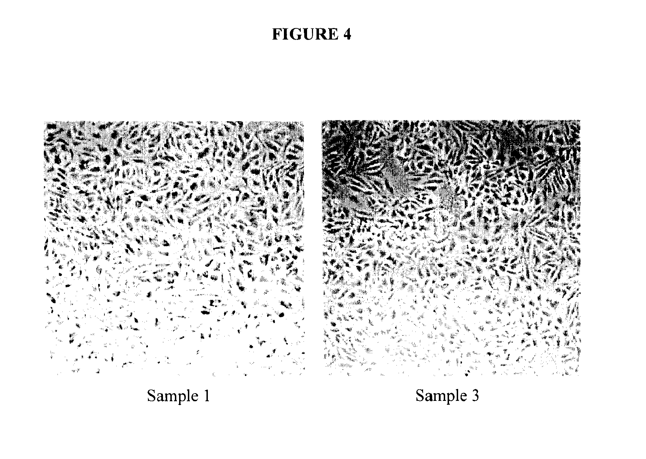

As shown in Fig. 4, 5X amplification of stained tissue culture from experiment 5 showing 39% bactofection efficiency from sample 1 and the lack of bactofection from sample 3.

| | | |
|---|---|---|
| Exp6: | 1 pYinv4 amplified MDS42RR trfA with arabinose | 30% |
| | 2 pYinv4 amplified MDS42RR trfA with arabinose | 30% |
| | 3 pYinv4 unamplified MDS42RR trfA | 0 |
| | 4 pYinv4 unamplified MDS42RR trfA | 0 |
| | 5 no plasmid MDS42RR trfA amplified trfA | 0 |
| | 6 no bacterial culture | 0 |
| | | |
| Exp7: | 1 pYinv4 amplified MDS42RR trfA with arabinose | >95% |
| | 2 pYinv4 amplified MDS42RR trfA with arabinose | >95% |
| | 3 pYinv4 unamplified MDS42RR trfA | <1% |
| | 4 pYinv4 unamplified MDS42RR trfA | <1% |
| | 5 no plasmid MDS42RR trfA amplified trfA | 0 |
| | 6 no bacterial culture | 0 |

### Example 7

### Reduced Vector/Host System

A reduced BAC vector/host system will be constructed in which the repE, parA and parB genes will be removed from the vector and provided by the host strain. The reduced BAC vector, called quarterbac, will be constructed by PCR amplifying the region of plasmid pKG15 or derivatives from oriS counterclockwise through parC. This plasmid will contain parC for partitioning, the unique SalI site flanked by transcriptional terminators on both sides for cloning, a drug resistance gene and both oriS and oriV origin of replication sequences but not their corresponding replication proteins trfA and repE. The quarterbac plasmid with two incomplete, non functional origins of replicatioin will be maintained in MDS42trfA in the presence of arabinose to allow replication from the oriV origin of replication.

The host strain will be constructed to contain the repE gene product to allow function of the oriS and the parA and parB gene products for stable partitioning of the low copy plasmid. The region from repE, including its promoter, through parA and parB from pKG15 will be amplified. Regions of homology to the E. coli chromosome will be added to the repE, parA, parB fragment and used for homologous recombination into the chromosome.

The quarterbac vector will be isolated from MDS42trfA and used as a selection for homologous recombination of the functional repE, parA and parB genes into the chromosome. In the absence of trfA, the oriV will not be active, similarly, repE gene product is required for oriS function. Stable resistance to the drug marker on the quarterbac vector (Cam^{R}) will indicate an active repE cell line and thus a candidate host strain. This cell line will be analyzed for correct insertion of the complete repE, parA, parB fragment.

### EMBODIMENTS

1. A vector comprising
   (a) an excisable fragment comprising an insertion site;
   (b) a first and second origin of replication; and
   (c) a pair of transcriptional terminators flanking the excisable fragment.
2. The vector of embodiment 1 wherein the first origin of replication is a low-copy number origin of replication.
3. The vector of embodiment 2 wherein the low-copy number origin of replication is *oriS.*
4. The vector of embodiment 1 wherein the second origin of replication is an inducible high-copy number origin of replication.
5. The vector of embodiment 4 wherein the high-copy number origin of replication is *oriV.*
6. The vector of embodiment 4 wherein the high-copy number origin of replication is under the control of an arabinose promoter.
7. The vector of embodiment 6 wherein the high-copy number origin of replication is regulated by a TrfA encoded by a gene under the control of an arabinose promoter
8. The vector of embodiment 1, wherein the excisable fragment comprises at least one type IIS restriction enzyme recognition site.
9. The vector of embodiment 1 , wherein the vector further comprises two inducible excision-mediating sites flanking the excisable fragment.
10. The vector of embodiment 4 wherein the excisable fragment comprises the second origin of replication.
11. The vector of embodiment 10 wherein the excisable fragment does not comprises the first origin of replication.
12. The vector of embodiment 4 wherein the excisable fragment does not comprise the first or second origin of replication.
13. The vector of embodiment 1, wherein the vector further comprises sequence primer binding sites flanking the excisable fragment.
14. A host cell comprising the vector of embodiment 1.
15. A method of cloning a heterologous nucleic acid comprising:
   (a) providing the heterologous nucleic acid;
   (b) providing a vector according to embodiment 1; and
   (c) introducing the heterologous nucleic acid into the insertion site of the vector.
16. The method of embodiment 15 wherein the vector has been digested with a TypeII S restriction enzyme, wherein the heterologous nucleic acid comprises blunt ends; wherein a double stranded adapter is further provided, and wherein a first end of the adapter is blunt and the second end of the adapter is complementary to the ends of the digested vector.
17. A vector produced by the method of embodiment 15.
18. The vector of embodiment 17 wherein the heterologous nucleic acid encodes a polypeptide.
19. The vector of embodiment 18 wherein the vector further comprises a promoter operatively linked to the heterologous nucleic acid.
20. The vector of embodiment 1 further comprising a heterologous nucleic acid.
21. A method of producing a library of nucleic acids comprising,
   (a) providing a library of heterologous nucleic acids;
   (b) providing a vector according to embodiment 1; and
   (c) introducing the heterologous nucleic acids into the insertion site of the vector.
22. The method of embodiment 21 wherein the vector has been digested with a TypeII S restriction enzyme, wherein the library of heterologous nucleic acids comprises blunt ends; wherein a double stranded adapter is further provided, and wherein a first end of the adapter is blunt and the second end of the adapter is complementary to the ends of the digested vector.
23. A library produced by the method of embodiment 21.
24. A method of inducing and expressing nucleic acid in an animal cell, the method comprising:
   (a) providing a vector comprising an insertion site, a first origin of replication a second origin of replication, and a pair of transcriptional terminators;
   (b) introducing the nucleic acid into the insertion site of the vector;
   (c) transforming at least one invasive bacterium with the vector to form at least one transformed bacterium; and
   (d) infecting the animal cell with said at least one transformed bacterium.
25. The method of embodiment 24 wherein the first origin of replication is a low-copy number origin of replication.
26. The method of embodiment 25 wherein the low-copy number origin of replication is *oriS.*
27. The method of embodiment 24 wherein the second origin of replication is an inducible high-copy number origin of replication.
28. The method of embodiment 27 wherein the high-copy number origin of replication is *oriV.*
29. The method of embodiment 28 wherein the high-copy number origin of replication is under the control of an arabinose promoter.
30. The method of embodiment 29 wherein the high-copy number origin of replication is regulated by a TrfA encoded by a gene under the control of an arabinose promoter
31. The method of embodiment 24 wherein the nucleic acid comprises heterologous DNA or RNA.
32. The method of embodiment 31 wherein heterologous DNA or RNA encodes a therapeutic or prophylactic agent.
33. The method of embodiment 32 where the therapeutic or prophylactic agent comprises an immunoregulatory agent, an antigen, antisense RNA, catalytic RNA, a protein, a polypeptide, an antibody, a cytokine, or a small molecule.
34. The method of embodiment 24, wherein said animal cells are mammalian cells.
35. The method of embodiment 34, wherein said mammalian cells are selected from the group consisting of human, bovine, ovine, porcine, feline, buffalo, canine, goat, equine, donkey, deer, and primate cells.
36. The method of embodiment 35, wherein said mammalian cells are human cells.
37. The method of embodiment 24, wherein said at least one bacterium are selected from the group consisting of Shigella spp, Listeria spp., Rickettsia spp and enteroinvasive Escherichia coli.
38. The method of embodiment 37, wherein said at least one bacterium has a reduced genome.
39. The method of embodiment 38, wherein said at least one bacterium is Escherichia coli.
40. The method of embodiment 39, wherein said said at least one bacterium is attenuated.
41. The method of embodiment 31, wherein said heterologous DNA or RNA encodes a member selected from the group consisting of a therapeutic protein, a small molecule, an immunoregulatory molecule, antisense RNA, and catalytic RNA.
42. The method of embodiment 41 wherein said member is expressed at least at a detectable level.
43. A method of inducing and expressing heterologous DNA or RNA in an animal cell, the method comprising:
   (a) providing a vector comprising an insertion site, a low-copy number origin of replication, an inducible high-copy number origin of replication, and a pair of transcriptional terminators;.
   (b) introducing a heterologous DNA or RNA into the insertion site of the vector;
   (c) transforming reduced genome *E*. *coli* with the vector to form at least one transformed E. coli; and
   (d) infecting the animal cell with said transformed *E*. *coli.*
44. The method of embodiment 43 where the *E*. *coli* is attenuated.
45. A reduced vector-host system comprising a host strain comprising repE, par A and parB genes and a vector that is free of repE, parA and parB genes.

## Claims

1. A method of inducing and expressing nucleic acid in an animal cell, the method comprising:
(a) providing a vector comprising an insertion site, a first origin of replication a second origin of replication, and a pair of transcriptional terminators;
(b) introducing the nucleic acid into the insertion site of the vector;
(c) transforming at least one invasive bacterium with the vector to form at least one transformed bacterium; and
(d) infecting the animal cell with said at least one transformed bacterium.

2. The method of claim 1 wherein the first origin of replication is a low-copy number origin of replication and wherein the second origin of replication is an inducible high-copy number origin of replication.

3. The method of claim 1 wherein the nucleic acid comprises heterologous DNA or RNA.

4. The method of claim 3 wherein heterologous DNA or RNA encodes a therapeutic or prophylactic agent.

5. The method of claim 4 where the therapeutic or prophylactic agent comprises an immunoregulatory agent, an antigen, antisense RNA, catalytic RNA, a protein, a polypeptide, an antibody, a cytokine, or a small molecule.

6. The method of claim 1, wherein said animal cells are mammalian cells.

7. The method of claim 6, wherein said mammalian cells are selected from the group consisting of human, bovine, ovine, porcine, feline, buffalo, canine, goat, equine, donkey, deer, and primate cells.

8. The method of claim 7, wherein said mammalian cells are human cells.

9. The method of claim 1, wherein said at least one bacterium are selected from the group consisting of Shigella spp, Listeria spp., Rickettsia spp and enteroinvasive Escherichia coli.

10. The method of claim 9, wherein said at least one bacterium has a reduced genome.

11. A method of inducing and expressing heterologous DNA or RNA in an animal cell, the method comprising:
(a) providing a vector comprising an insertion site, a low-copy number origin of replication, an inducible high-copy number origin of replication, and a pair of transcriptional terminators;.
(b) introducing a heterologous DNA or RNA into the insertion site of the vector;
(c) transforming reduced genome *E*. *coli* with the vector to form at least one transformed E. coli; and
(d) infecting the animal cell with said transformed *E*. *coli.*

12. A vector comprising
(a) an excisable fragment comprising an insertion site;
(b) a first and second origin of replication; and
(c) a pair of transcriptional terminators flanking the excisable fragment.

13. A host cell comprising the vector of claim 12.

14. A method of cloning a heterologous nucleic acid comprising:
(a) providing the heterologous nucleic acid;
(b) providing a vector comprising
(i) an excisable fragment comprising an insertion site;
(ii) a first and second origin of replication; and
(iii) a pair of transcriptional terminators flanking the excisable fragment; and
(c) introducing the heterologous nucleic acid into the insertion site of the vector.

15. A method of producing a library of nucleic acids comprising,
(a) providing a library of heterologous nucleic acids;
(b) providing a vector according to claim 12; and
(c) introducing the heterologous nucleic acids into the insertion site of the vector.


SEQUENCE LISTING

<110> Scarab Genomics, LLC

<120> VECTOR SYSTEMS

<130> 02730.0011.00PC02

<140> PCT/US2007/075214
<141> 2007-08-03

<150> 60/821,339
<151> 2006-08-03

<160> 5

<170> PatentIn version 3.5

<210> 1
<211> 10
<212> DNA
<213> Artificial

<220>
<223> Synthetic


<220>
<221> misc_feature
<222> (5)..(10)
<223> n is a, c, g, or t

<400> 1
gaagnnnnnn 10


<210> 2
<211> 20
<212> DNA
<213> Artificial

<220>
<223> Synthetic

<400> 2
cttcggcgcg ccgcggccgc 20


<210> 3
<211> 16
<212> DNA
<213> Artificial

<220>
<223> Synthetic

<400> 3
gcggccgcgg cgcgcc 16


<210> 4
<211> 21
<212> DNA
<213> Artificial

<220>
<223>  Synthetic

<400>  4
cttcggcgcg ccgcggccgc t                                                21


<210>  5
<211>  16
<212>  DNA
<213>  Artificial

<220>
<223>  Synthetic

<400>  5
gcggccgcgg cgcgcc                                                      16